# FASCICULE DE BREVET EUROPEEN

(11) **EP 3 853 341 B1**
(45) Date de publication et mention de la délivrance du brevet: **13.04.2022**
(21) Numéro de dépôt: 19770079.2
(22) Date de dépôt: 18.09.2019
(51) Int. Cl.: C12M 1/42, C12N 15/82

(54) **DISPOSITIF DE DISTRIBUTION DE MICROBULLES POUR UNE SONOPORATION CELLULAIRE**
VORRICHTUNG ZUR ABGABE VON MIKROKÜGELCHEN ZUR ZELLSONOPORATION
DEVICE FOR DISPENSING MICROBEADS FOR CELL SONOPORATION

(30) Priorité: 18.09.2018 FR 1858385
(43) Date de publication de la demande: 28.07.2021
(73) Titulaire: CENTRE NATIONAL DE LA RECHERCHE SCIENTIFIQUE (CNRS), 75016 Paris (FR); Université d'Orléans, 45000 Orléans (FR)
(72) Inventeur: DELALANDE, Anthony, 45640 SANDILLON (FR); LEBERTRE, Matthias, 37200 TOURS (FR); DELALANDE, Michaël, 38000 GRENOBLE (FR); PICHON, Chantal, 45500 SAINT DENIS DE L'HOTEL (FR)
(74) Mandataire: Regimbeau
(86) Numéro de dépôt international: PCT/EP2019/075075
(87) Numéro de publication internationale: WO 2020/058367

(56) Documents cités:
- US-A- 5 141 131
- US-A1- 2010 143 241

## Description

### DOMAINE DE L'INVENTION

La présente invention concerne le domaine de la transfection cellulaire, notamment la sonoporation à l'aide de microbulles. L'invention concerne plus particulièrement un dispositif de distribution de microbulles, ainsi qu'un procédé de sonoporation cellulaire au moyen d'un tel dispositif.

### ARRIERE-PLAN TECHNOLOGIQUE

La transfection cellulaire est une méthode consistant à introduire des molécules actives (telles que des acides nucléiques, molécules chimio-thérapeutiques, marqueurs, protéines, etc.) dans des cellules à transfecter, sans recourir à des virus, par opposition à la transduction.

La sonoporation cellulaire est une méthode de transfection consistant à utiliser des ondes ultrasonores dans le but de modifier la perméabilité des cellules, de façon à permettre l'introduction des molécules actives à l'intérieur des cellules à transfecter.

Un dispositif de sonoporation de type connu est par exemple décrit dans le document WO 2015/110955. Ce dispositif comprend un transducteur ultrasonore placé au fond d'une cuve remplie d'un liquide et émettant des ondes ultrasonores vers des cellules à transfecter placées sur une structure déplaçable.

De manière connue, le rendement de l'opération de sonoporation peut être grandement amélioré en encapsulant les molécules actives dans des microbulles. Les microbulles contenant les molécules actives sont alors mises en contact des cellules à transfecter et vibrent sous l'effet des ondes ultrasonores émises par le transducteur. Leurs interactions avec les cellules à transfecter perméabilisent les membranes de ces dernières, les molécules actives pouvant alors pénétrer à l'intérieur des cellules à transfecter.

Un problème est que la distribution des microbulles au-dessus des cellules à transfecter est mise en œuvre manuellement par un utilisateur au moyen d'une micropipette. Le document US 2010/143241 A1 décrit par exemple un dispositif de sonoporation cellulaire à l'aide de microbulles, comprenant une pipette. Une telle distribution est fastidieuse et conduit à un mauvais rendement de l'opération de sonoporation.

### RESUME DE L'INVENTION

Un but de l'invention est de proposer un dispositif de distribution de microbulles pour une sonoporation cellulaire présentant un rendement amélioré par rapport aux dispositifs de type connu.

Selon un premier aspect, l'invention concerne un dispositif de distribution de microbulles pour une sonoporation cellulaire, caractérisé en ce que le dispositif comprend :
- un tube symétrique autour d'un axe vertical, ledit tube étant configuré pour recevoir un flacon contenant une solution de microbulles,
- au moins deux cathéters disposés à l'intérieur du tube, lesdits cathéters étant au moins en partie orientés de façon sensiblement parallèle à l'axe vertical, chacun des cathéters présentant en une première extrémité un orifice adapté pour déboucher dans le flacon lorsque celui-ci est reçu par le tube, le premier cathéter étant adapté pour être raccordé à une première source d'injection d'air, le deuxième cathéter présentant une deuxième extrémité,
dans lequel, lorsque le premier cathéter est raccordé à la première source d'injection d'air, de l'air peut être injecté dans le flacon via l'orifice du premier cathéter, l'air injecté générant une surpression dans le flacon, de sorte que des microbulles contenues dans le flacon entrent dans le deuxième cathéter via l'orifice de sa première extrémité, puis se déplacent à l'intérieur du deuxième cathéter vers sa deuxième extrémité.

Un tel dispositif est capable de distribuer une quantité précise et maîtrisée de microbulles de façon automatique, sans nécessiter d'opération manuelle de la part de l'utilisateur. Le dispositif permet une précision améliorée de l'emplacement de la distribution des microbulles, les positions du tube et des cathéters étant fixes, contrairement au cas où l'utilisateur doit tenir la pipette contenant les microbulles à la main.

Un tel dispositif conduit donc à des rendements de distribution de microbulles améliorés.

Par ailleurs, une telle distribution des microbulles ne nécessite pas de contact avec les microbulles, ce qui permet, au cours d'une sonoporation, de préserver les cellules de toute contamination biologique.

Le dispositif de distribution de microbulles peut comprendre un troisième cathéter au moins en partie situé autour d'au moins une partie du deuxième cathéter, le troisième cathéter étant adapté pour être raccordé à une deuxième source d'injection d'air. Ce troisième cathéter permet, via la deuxième source d'injection d'air, de détacher les microbulles qui se sont déplacées jusqu'à la deuxième extrémité du deuxième cathéter pour les distribuer à des cellules à transfecter. Le dépôt des microbulles avec ce troisième cathéter est plus rapide et plus précis.

L'invention concerne également un dispositif de sonoporation cellulaire comprenant un tel dispositif de distribution de microbulles.

Le dispositif de sonoporation peut comprendre une première source d'injection d'air raccordée au premier cathéter. Cette première source d'injection d'air permet d'injecter de l'air dans le flacon reçu par le dispositif de distribution de microbulles, afin de faire entrer des microbulles à travers le deuxième cathéter et de les déplacer vers la deuxième extrémité du deuxième cathéter.

Le dispositif de sonoporation peut éventuellement comprendre une deuxième source d'injection d'air raccordée au troisième cathéter, lorsque le dispositif de distribution de microbulles comprend un troisième cathéter. Ainsi, les microbulles qui se sont déplacées jusqu'à la deuxième extrémité du deuxième cathéter peuvent être détachées de celle-ci de manière automatique et précise, pour être distribuées aux cellules à transfecter.

Le dispositif de sonoporation cellulaire peut comprendre en outre :
- une cuve destinée à être remplie au moins partiellement d'un liquide,
- un transducteur ultrasonore disposé à l'intérieur de la cuve,
- une plaque comprenant au moins un logement destiné à recevoir des cellules à transfecter,
- un support mobile adapté pour recevoir la plaque,
- une unité de déplacement du support mobile, configurée pour déplacer le support.

Avec un tel dispositif de sonoporation cellulaire, les cellules à transfecter peuvent être déplacées par rapport au dispositif de distribution de microbulles et au transducteur ultrasonore. La précision de la distribution des microbulles est donc améliorée, ce qui contribue à améliorer les rendements de sonoporation par rapport aux dispositifs connus.

Certaines caractéristiques préférées mais non limitatives du dispositif de sonoporation cellulaire sont les suivantes, prises individuellement ou en combinaison :
- l'unité de déplacement comprend des rails s'étendant dans au moins deux dimensions définissant un plan perpendiculaire à l'axe vertical, dit plan horizontal, la troisième dimension étant celle de l'axe vertical, le support étant destiné à être déplacé le long desdits rails. Ainsi, l'emplacement de la plaque contenant les cellules à transfecter vis-à-vis du dispositif de sonoporation peut être contrôlé, ce qui améliore la précision de l'emplacement des microbulles distribuées.
- l'unité de déplacement comprend en outre un moteur adapté pour déplacer le support dans au moins deux dimensions définissant un plan perpendiculaire à l'axe vertical, dit plan horizontal, la troisième dimension étant celle de l'axe vertical. Ainsi, l'emplacement de la plaque contenant les cellules à transfecter par rapport à celui du dispositif de sonoporation peut être contrôlé de manière automatique, sans nécessiter d'opération manuelle de la part d'un utilisateur.
- le dispositif de distribution de microbulles est positionné en face du transducteur ultrasonore selon l'axe vertical. Ainsi, les cellules à transfecter peuvent être déplacées par rapport à la fois au transducteur ultrasonore et au dispositif de distribution de microbulles, et peuvent être placées sur le même axe que ces deux éléments, ce qui améliore le rendement de l'opération de sonoporation.
- le dispositif de sonoporation comprend en outre un boîtier comprenant une première partie configurée pour loger les éventuelles sources d'injection d'air ainsi qu'au moins une partie de l'unité de déplacement, le boîtier comprenant en outre une deuxième partie configurée pour loger le dispositif de distribution de microbulles, la cuve, le transducteur ultrasonore, la plaque et le support mobile, la deuxième partie comprenant en outre un capot adapté pour être ouvert ou fermé, ledit capot permettant en position ouverte à un utilisateur d'accéder au moins à la plaque. Un tel boîtier peut permettre de contenir l'ensemble du dispositif de sonoporation cellulaire dans un PSM, et d'améliorer sa modularité. En position fermée, le capot limite l'évaporation, isole les cellules à transfecter et protège les utilisateurs du mouvement du support. En position ouverte, un utilisateur peut accéder à certains éléments du dispositif, qui peuvent ainsi être changés de façon modulaire et/ou nettoyés, la maintenance d'un tel dispositif étant facilitée.

Le dispositif de sonoporation peut en outre comprendre une unité de traitement configurée pour contrôler au moins l'un des éléments suivants : dispositif de distribution de microbulles, transducteur ultrasonore, unité de déplacement, éventuelles première source d'injection d'air et deuxième source d'injection d'air, capot. Une telle unité de traitement permet un contrôle automatique de la séquence de distribution de microbulles, du déplacement de la plaque, des caractéristiques des ondes ultrasonores, ainsi que des paramètres de l'opération de sonoporation.

Selon un deuxième aspect, l'invention concerne un procédé de sonoporation cellulaire au moyen d'un dispositif de distribution de microbulles selon le premier aspect, un flacon contenant une solution de microbulles étant reçu dans le dispositif de distribution de microbulles, le procédé de sonoporation comprenant une étape d'injection d'air dans le flacon via l'orifice de la première extrémité du premier cathéter, l'air injecté générant une surpression dans le flacon, de sorte que des microbulles contenues dans le flacon entrent dans le deuxième cathéter via l'orifice de sa première extrémité puis se déplacent à l'intérieur du deuxième cathéter vers sa deuxième extrémité.

Le procédé de sonoporation peut comprendre en outre les étapes suivantes, prises individuellement ou en combinaison :
- une étape d'injection d'air dans un troisième cathéter au moins en partie situé autour d'au moins une partie du deuxième cathéter,
- une étape de déplacement du support dans laquelle, le dispositif de distribution de microbulles étant positionné en face du transducteur ultrasonore selon l'axe vertical, le support est déplacé de manière à placer successivement chaque logement de la plaque entre le dispositif de distribution de microbulles et le transducteur ultrasonore selon l'axe vertical.

Selon un troisième aspect, l'invention concerne un produit programme d'ordinateur comprenant des instructions de code pour l'exécution d'un procédé de sonoporation cellulaire lorsque ce programme est exécuté par un processeur.

### PRÉSENTATION DES FIGURES

D'autres aspects, buts et avantages de la présente invention apparaîtront à la lecture de la description détaillée qui va suivre, donnée à titre d'exemple non limitatif, qui sera illustrée par les figures suivantes :
- Les figures 1a et 1b sont des schémas représentant un dispositif de distribution de microbulles conforme à l'invention.
- La figure 2 est un schéma représentant une cuve et un transducteur ultrasonore pour un dispositif de sonoporation conforme à l'invention.
- Les figures 3a et 3b sont des schémas représentant un dispositif de sonoporation conforme à l'invention, respectivement en positions fermée et ouverte du capot.
- La figure 4 est un schéma représentant un dispositif de sonoporation conforme à l'invention.
- La figure 5a est un schéma représentant un procédé de sonoporation conforme à l'invention.
- La figure 5b est un schéma représentant une séquence de déplacement et de distribution de microbulles conforme à l'invention.

### DESCRIPTION DETAILLEE D'UN MODE DE RÉALISATION

### Dispositif de distribution de microbulles et de sonoporation

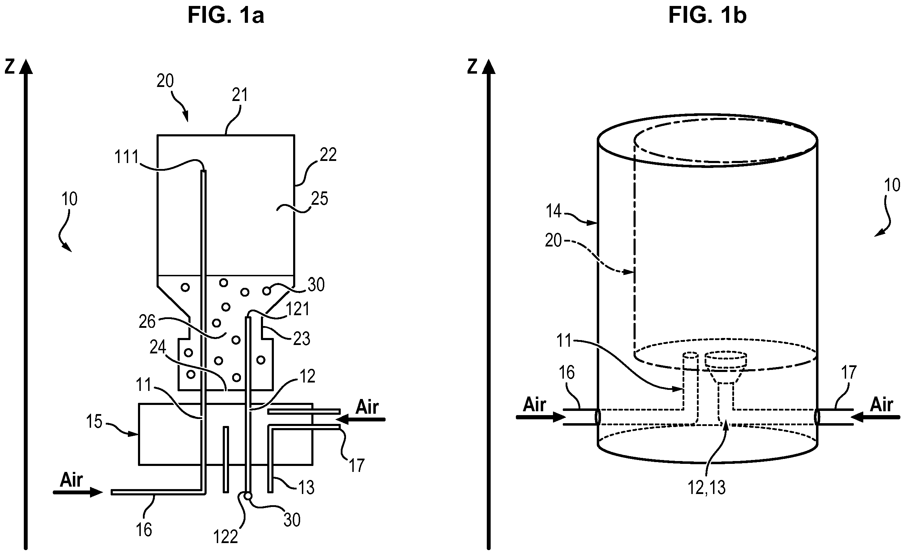

Les figures 1a et 1b illustrent un dispositif 10 de distribution de microbulles 30 pour une sonoporation cellulaire.

Le dispositif 10 de distribution de microbulles 30 comprend un tube 14. Le tube 14 peut être rigide, de forme sensiblement cylindrique, et présenter une symétrie de rotation par rapport à un axe Z vertical.

Le tube 14 définit un espace adapté pour recevoir un flacon 20 contenant une solution 26 de microbulles 30. Le diamètre des microbulles 30 peut varier en fonction du type de sonoporation à réaliser.

Le flacon 20 peut être un flacon de type flacon à sertir. Le flacon 20 peut comporter un fond 21, un corps 22 sensiblement cylindrique, un col 23 de dimensions plus faibles que le corps 22, et une tête 24 élargie par rapport au col 23, la tête 24 étant de préférence refermée par un septum.

Le flacon 20 est de préférence positionné tête 24 vers le bas dans le tube 14 du dispositif 10 de distribution de microbulles 30. Ainsi positionné, la tête 24 du flacon 20, son col 23 et une partie de son corps 22 sont remplis de la solution 26 contenant les microbulles 30. La partie du flacon 20 qui n'est pas remplie par la solution 26 est remplie d'air 25. Le corps 22 du flacon 20 peut être directement en contact avec le tube 14 du dispositif 10 de distribution de microbulles 30. Dans une variante, un espace subsiste entre le corps 22 et le tube 14.

Le dispositif 10 de distribution de microbulles 30 peut également présenter une partie inférieure 15 cylindrique. La partie inférieure 15 présente une symétrie de rotation par rapport à l'axe Z vertical passant par le centre du tube 14, et peut être en contact avec le tube 14. La partie inférieure 15 peut présenter un diamètre supérieur à celui du tube 14 destiné à recevoir le flacon 20.

Le dispositif 10 de distribution de microbulles 30 comprend un premier cathéter 11. Le premier cathéter 11 est fixé dans la partie inférieure 15 du dispositif 10 de distribution de microbulles 30. Le premier cathéter 11 peut être de forme sensiblement cylindrique et présenter une symétrie de rotation autour de l'axe Z vertical.

Le premier cathéter 11 présente une première extrémité 111, qui présente un orifice. Lorsque le flacon 20 est reçu dans le tube 14, le premier cathéter 11 perce le septum du flacon 20 et l'orifice de sa première extrémité 111 débouche à la verticale dans le flacon 20, de préférence dans l'air 25 contenu dans le flacon 20.

Le premier cathéter 11 est adapté pour être raccordé à une première source d'injection d'air. Le raccord entre la première source d'injection d'air et le premier cathéter 11 est effectué par un premier tuyau d'arrivée d'air 16.

Ainsi, l'air injecté par la première source d'injection d'air traverse le premier tuyau d'arrivée d'air 16 puis le premier cathéter 11, et est injecté dans le flacon 20 via l'orifice de la première extrémité 111 du premier cathéter 11, ce qui engendre une surpression dans le flacon 20.

Le dispositif 10 de distribution de microbulles 30 comprend un deuxième cathéter 12. Le deuxième cathéter 12 est fixé dans la partie inférieure 15 du dispositif 10 de distribution de microbulles 30. Le deuxième cathéter 12 peut être de forme sensiblement cylindrique et présenter une symétrie de rotation autour de l'axe Z vertical.

Le deuxième cathéter 12 présente une première extrémité 121, qui présente un orifice. Lorsque le flacon 20 est reçu dans le tube 14, le deuxième cathéter 12 perce le septum du flacon 20 et l'orifice de sa première extrémité 121 débouche à la verticale dans le flacon 20, de préférence dans la solution 26 de microbulles 30 contenue dans le flacon 20.

Le deuxième cathéter 12 présente une deuxième extrémité 122. La deuxième extrémité 122 du deuxième cathéter 12 débouche de préférence à l'extérieur du tube 14 et de la partie inférieure 15 du dispositif 10 de distribution de microbulles 30.

Ainsi, la surpression générée dans le flacon 20 pousse les microbulles 30 contenues dans le flacon 20 à entrer dans le deuxième cathéter 12 via l'orifice de sa première extrémité 121, puis à se déplacer à l'intérieur du deuxième cathéter 12 vers sa deuxième extrémité 122.

Selon un mode de réalisation préféré, le dispositif 10 de distribution de microbulles 30 comprend un troisième cathéter 13. Le troisième cathéter 13 peut être de forme sensiblement cylindrique et présenter une symétrie de rotation autour de cet axe Z vertical.

Le troisième cathéter 13 est situé autour d'au moins une partie du deuxième cathéter 12. Le troisième cathéter peut être fixé et situé à l'intérieur de la partie inférieure 15 du dispositif 10 de distribution de microbulles 30. Une extrémité du troisième cathéter 13 peut déboucher à l'extérieur du tube 14 et de la partie inférieure 15 du dispositif 10 de distribution de microbulles 30, et à proximité de la deuxième extrémité 122 du deuxième cathéter 12.

Le troisième cathéter 13 est adapté pour être raccordé à une deuxième source d'injection d'air. Le raccord entre la deuxième source d'injection d'air et le troisième cathéter 13 est effectué par un deuxième tuyau d'arrivée d'air 17.

Ainsi, de l'air peut être injecté par la deuxième source d'injection d'air dans le troisième cathéter 13, l'air injecté détachant les microbulles 30 qui se sont déplacées jusqu'à la deuxième extrémité 122 du deuxième cathéter 12 pour les distribuer à des cellules à transfecter.

Selon un mode de réalisation préféré, le dispositif 10 de distribution de microbulles 30 est fixe. En variante, le dispositif 10 de distribution de microbulles 30 peut comprendre une unité de déplacement permettant de le déplacer, notamment en translation suivant une ou plusieurs directions. Un tel déplacement du dispositif 10 de distribution de microbulles 30 participe à la précision de la localisation de la distribution des microbulles 30.

Le dispositif 10 de distribution de microbulles 30 peut être utilisé avec des microbulles 30 de type anionique, qui sont les microbulles 30 les plus couramment utilisées à l'heure actuelle pour réaliser des opérations de sonoporation cellulaire.

En variante, le dispositif 10 de distribution de microbulles 30 peut être utilisé avec des microbulles 30 de type cationique. De telles microbulles 30 cationiques sont capables d'interactions électrostatiques avec les molécules actives telles que les acides nucléiques, qui sont anioniques et se fixent alors à l'extérieur des microbulles 30. Ces microbulles 30 cationiques sont obtenues en ajoutant des lipides aux microbulles 30 anioniques. Elles permettent des rendements de sonoporation de l'ordre de cinq fois supérieurs aux rendements obtenus avec des microbulles 30 anioniques.

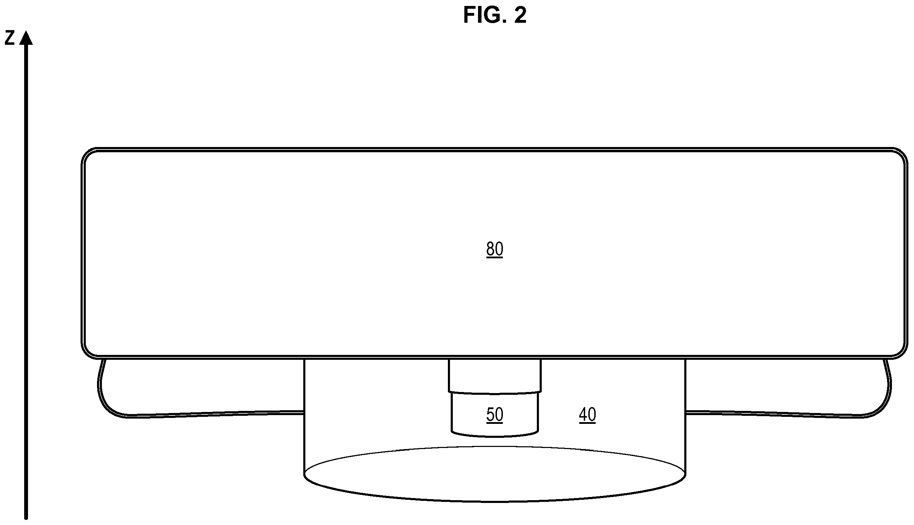

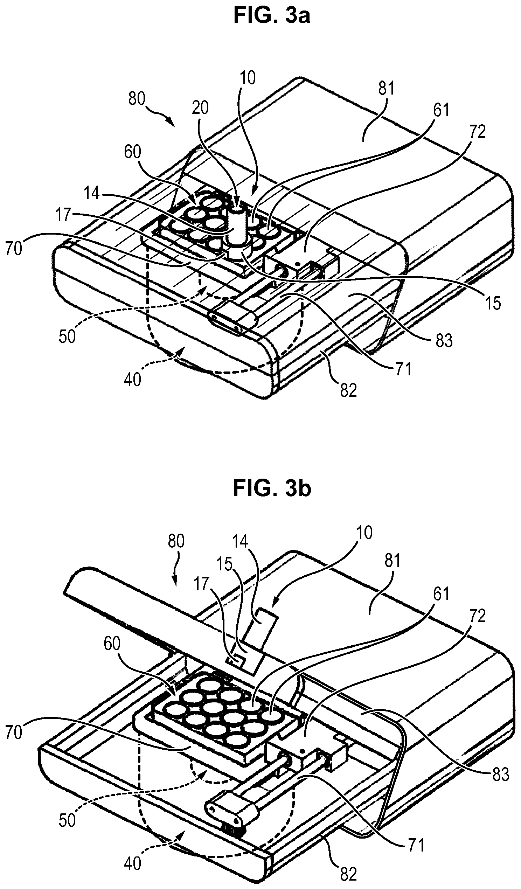

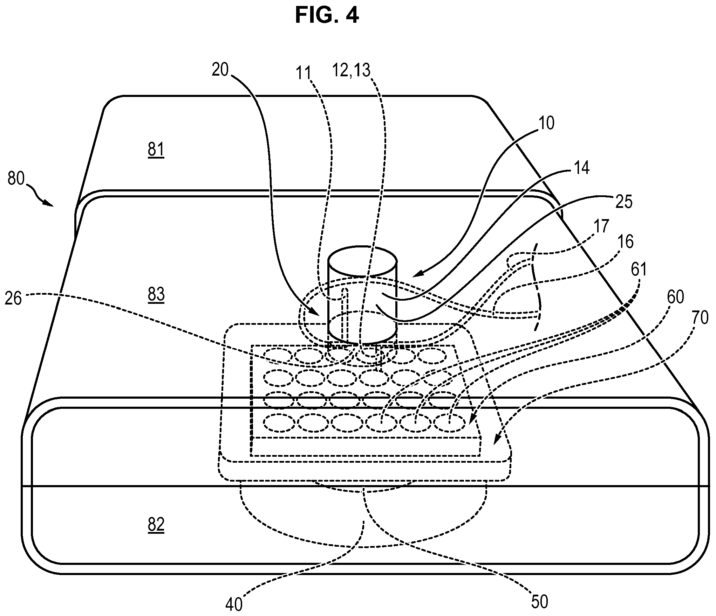

Les figures 2, 3a, 3b et 4 représentent des dispositifs de sonoporation cellulaire comprenant des dispositifs de distribution de microbulles 10 selon un mode de réalisation.

Le dispositif de sonoporation peut également comprendre une première source d'injection d'air raccordée au premier cathéter 11. Le dispositif de sonoporation peut également comprendre une deuxième source d'injection d'air raccordée au troisième cathéter 13, lorsque le dispositif 10 de distribution de microbulles 30 comprend un troisième cathéter 13. Les première et deuxième sources d'injection d'air sont par exemple des pompes ou des compresseurs.

Selon un mode de réalisation préféré, le dispositif de sonoporation cellulaire comprend en outre :
- une cuve 40 destinée à être remplie au moins partiellement d'un liquide,
- un transducteur ultrasonore 50 disposé à l'intérieur de la cuve 40,
- une plaque 60 comprenant au moins un logement 61 destiné à recevoir des cellules à transfecter,
- un support 70 mobile adapté pour recevoir la plaque 60,
- une unité de déplacement 71, 72 du support 70 configurée pour déplacer le support 70.

Selon un mode de réalisation préféré, la cuve 40 est de forme sensiblement cylindrique autour de l'axe Z vertical. La cuve 40 peut être translucide et être remplie d'un liquide, tel que de l'eau, permettant de propager les ondes ultrasonores en provenance du transducteur 50. La taille de la cuve 40 est adaptée à l'encombrement de la plaque 60 et du support 70. La cuve 40 peut également comprendre des moyens de détection du niveau de liquide, capables de vérifier que le niveau de liquide est suffisant pour permettre la bonne propagation des ondes ultrasonores dans la cuve 40. La cuve 40 peut également comprendre un robinet de vidange permettant de vidanger le liquide qu'elle contient. Par ailleurs, la cuve 40 peut contenir un joint d'étanchéité, par exemple de type double joint torique, afin d'assurer l'étanchéité entre le transducteur ultrasonore 50 et le liquide qu'elle contient.

Le transducteur ultrasonore 50 est de préférence fixe et situé au centre de la cuve 40. Il peut être situé sensiblement au milieu de celle-ci. Le transducteur ultrasonore 50 est relié à une source de puissance par un câble débouchant à travers un orifice de la cuve 40. Le transducteur ultrasonore 50 peut être interchangé, afin d'adapter les caractéristiques des ondes ultrasonores en fonction par exemple de la taille des microbulles 30 utilisées, de la nature des molécules actives et/ou des cellules à transfecter, du rendement et/ou de la vitesse de l'opération de sonoporation souhaités, etc.

La plaque 60 s'étend de préférence selon un plan perpendiculaire à l'axe Z vertical, dit plan horizontal. La plaque 60 peut présenter une face inférieure et une face supérieure sensiblement rectangulaires et de mêmes dimensions. Les dimensions de la plaque 60 peuvent être adaptées pour pouvoir être contenue dans l'enceinte d'un PSM. La plaque 60 peut comporter plusieurs logements 61 répartis de façon régulière sur sa face supérieure, les logements 61 étant destinés à contenir des cellules à transfecter. Les logements 61 peuvent être cylindriques, et s'étendre selon l'axe Z vertical dans la profondeur de la plaque 60. La distance entre la face inférieure de la plaque 60 et le fond des logements 61 peut être de l'ordre du millimètre. La plaque 60 peut être placée entre le transducteur ultrasonore 50 et le dispositif 10 de distribution de microbulles 30 selon l'axe Z vertical. Le transducteur ultrasonore 50 peut être placé du côté de la face inférieure de la plaque 60, de préférence à une distance fixe par rapport à celle-ci. Le dispositif 10 de distribution de microbulles 30 peut être placé du côté de sa face supérieure, de préférence à une distance fixe par rapport à celle-ci.

Le support 70 peut présenter des dimensions cohérentes de la plaque 60 et s'étendre tout autour de celle-ci. Le support 70 peut fixer la plaque 60 par exemple par encastrement, ou par des moyens de fixations tels que des vis.

L'unité de déplacement 71, 72 peut comprendre des rails de déplacement 71 s'étendant dans au moins deux dimensions définissant un plan perpendiculaire à l'axe Z vertical, dit plan horizontal, la troisième dimension étant celle de l'axe Z vertical. L'unité de déplacement 71, 72 peut également comprendre un élément de déplacement 72, adapté pour coulisser le long des rails 71. Le support 70 mobile peut être fixé à l'élément de déplacement 72, et déplacé le long des rails 71 en translation dans le plan horizontal, et le cas échéant selon l'axe Z vertical.

L'unité de déplacement 71, 72 peut comprendre en outre un ou plusieurs moteurs adaptés pour déplacer le support 70 dans au moins une des dimensions définies précédemment. Ainsi, le déplacement du support 70 peut se faire de façon automatique par des moteurs. En variante, l'unité de déplacement 71, 72 peut comporter des moyens manuels de déplacement, par exemple des vis micrométriques. En variante, l'unité de déplacement peut comporter à la fois des moteurs et des moyens manuels de déplacement. Par exemple, le support 70 peut être déplacé automatiquement par des moteurs dans le plan horizontal, et manuellement à l'aide de vis micrométriques le long de l'axe Z vertical.

Selon un mode de réalisation préféré, le dispositif 10 de distribution de microbulles 30 est positionné en face du transducteur ultrasonore 50 selon l'axe Z vertical. L'unité de déplacement 71, 72 peut alors déplacer le support 70, et donc la plaque 60, de façon à placer successivement chaque logement 61 entre le transducteur ultrasonore 50 et le dispositif 10 de distribution de microbulles 30 sur l'axe Z vertical. Les microbulles 30 une fois détachées de la deuxième extrémité 122 du deuxième cathéter 12 peuvent alors être distribuées aux logements 61 sous l'effet de la gravité.

Dans le dispositif de sonoporation décrit dans les paragraphes précédents, le flacon 20 est situé à proximité de la deuxième extrémité 122 du deuxième cathéter 12, qui est elle-même située à proximité des logements 61 contenant les cellules à transfecter. Un tel dispositif permet de limiter la distance à parcourir par les microbulles 30 entre le flacon 20 et les cellules à transfecter, et notamment de limiter les dimensions du deuxième cathéter 12, ce qui conduit à des rendements de sonoporation plus élevés.

En variante, le flacon 20 contenant les microbulles 30 peut être positionné de façon plus éloignée des cellules à transfecter, par exemple sur un côté du dispositif.

Le dispositif de sonoporation peut également comprendre une source de puissance capable d'alimenter le transducteur ultrasonore 50.

Selon un mode de réalisation préféré, le dispositif de sonoporation comprend également un boîtier 80. Le tube 14 du dispositif 10 de distribution de microbulles 30 peut être fixé au boîtier 80 et en dépasser. Ainsi, l'introduction du flacon 20 dans le tube 14 peut être effectuée depuis l'extérieur du boîtier 80. Dans le cas où le dispositif 10 de distribution de microbulles 30 comprend une unité de déplacement, cette unité de déplacement peut par exemple comprendre des rails de déplacement fixés au boîtier 80, le long desquels le dispositif 10 de distribution de microbulles 30 peut se déplacer. Le tube 14 du dispositif 10 de distribution de microbulles 30 peut être alors fixé aux rails et non au boîtier 80.

En variante, certains éléments, par exemple la cuve 40 et le transducteur ultrasonore 50, ou encore le dispositif 10 de distribution de microbulles 30, peuvent être situés à l'extérieur du boîtier 80.

Selon un mode de réalisation préféré, le boîtier 80 comprend une première partie 81 et une deuxième partie 82. La première partie 81 peut être opaque et la deuxième partie 82 peut être transparente. La première partie 81 peut comprendre les deux sources d'injection d'air, la source de puissance, le moteur de l'unité de déplacement 71, 72, ainsi qu'une partie ou l'ensemble des rails de déplacement 71. La deuxième partie 82 peut comprendre le dispositif 10 de distribution de microbulles 30, la cuve 40, le transducteur ultrasonore 50, la plaque 60 et son support 70, ainsi qu'une partie ou l'ensemble des rails de déplacement 71.

La deuxième partie 82 du boîtier 80 peut également comprendre un capot 83 adapté pour être ouvert ou fermé. Le capot 83 peut être transparent. En position fermée, le capot 83 limite l'évaporation, isole les cellules à transfecter et protège les utilisateurs du mouvement du support 70. En position ouverte, un utilisateur peut accéder à certains éléments du dispositif, tels que la plaque 60, le support 70, la cuve 40 et son transducteur 50. Ainsi, certains éléments peuvent être changés de façon modulaire et/ou nettoyés, ce qui facilite la maintenance d'un tel dispositif. De préférence, le capot 83 comprend des moyens de fermeture adaptés pour être actionnés de manière automatique.

Selon un mode de réalisation préféré, le dispositif de sonoporation cellulaire comprend en outre une unité de traitement configurée pour contrôler à l'aide d'un logiciel au moins l'un des éléments suivants : dispositif 10 de distribution de microbulles 30, transducteur ultrasonore 50, unité de déplacement 71, 72, première source d'injection d'air et deuxième source d'injection d'air, moyens de fermeture du capot 83.

L'unité de traitement peut être située dans ou à l'extérieur du boîtier 80 de sonoporation. Elle peut être reliée par exemple par liaison USB à un ordinateur qui comporte une interface homme-machine permettant à un utilisateur de contrôler l'opération de sonoporation.

Une telle unité de traitement contrôle de manière automatique les caractéristiques de la distribution des microbulles 30 (volume distribué, position et instant de distribution, temps entre chaque microbulle 30 distribuée, nombre de distributions, etc.), les paramètres des ondes ultrasonores (fréquence, amplitude, puissance, durée de transmission, etc.), le déplacement du support 70 (directions et vitesse de déplacement, position), l'ouverture et la fermeture du capot 83, etc.

Le dispositif de sonoporation décrit dans les paragraphes ci-dessus est destiné à être utilisé in vitro. En variante, le dispositif peut être utilisé in vivo, l'opération de sonoporation étant contrôlée avec la même unité de traitement que pour une utilisation in vitro. Le transducteur ultrasonore 50 est alors choisi de manière à être compatible des amplitudes nécessaires à une utilisation in vivo, qui sont supérieures à celles d'une utilisation in vitro. La cuve 40 peut choisie de sorte à ce que ses dimensions soient plus petites que la cuve 40 d'une sonoporation in vitro, et est placée sur un tissu à observer, par exemple la peau d'un animal. Les microbulles 30 sont injectées dans le sang par intraveineuse.

### Procédé de sonoporation

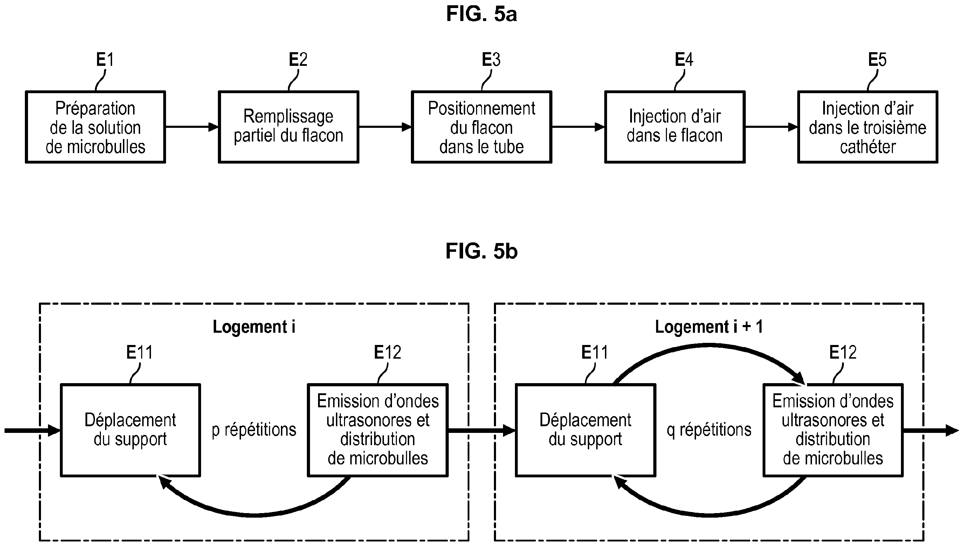

Un procédé de sonoporation au moyen d'un dispositif 10 de distribution de microbulles 30 tel que décrit dans les paragraphes précédents est illustré en figure 5a.

Selon une première étape E1, la solution 26 contenant les microbulles 30 est préparée. Cette étape E1 comprend la réalisation de microbulles 30 anioniques ou cationiques, la mise en place des molécules actives au sein de ces microbulles 30 et la préparation de la solution 26 les contenant.

Selon une deuxième étape E2, le flacon 20 est rempli au moins partiellement de la solution 26 contenant les microbulles 30. Le flacon 20 peut être agité au cours de cette étape E2. Le niveau de remplissage du flacon 20 peut dépendre de la quantité de molécules actives à transfecter, du nombre de logements 61 présents sur la plaque 60 et du nombre de cellules à transfecter, des dimensions des cathéters 11, 12, 13, etc.

Selon une troisième étape E3, le flacon 20 est positionné dans le tube 14 du dispositif 10 de distribution de microbulles 30, tête 24 vers le bas. Le septum du flacon 20 est alors percé par les premier et deuxième cathéters 11, 12.

Selon une quatrième étape E4, de l'air est injecté par la première source d'injection d'air. L'air injecté traverse le premier tuyau d'arrivée d'air 16 et le premier cathéter 11, et débouche dans l'air 25 contenu dans le flacon 20 à travers l'orifice de la première extrémité 111 du premier cathéter 11. Le volume d'air injecté est de l'ordre du microlitre. L'air ainsi injecté génère une surpression de l'air 25 contenu dans le flacon 20. Cette surpression amène les microbulles 30 contenues dans la solution 26 à entrer dans le deuxième cathéter 12 via l'orifice de sa première extrémité 121, puis à se déplacer à l'intérieur du deuxième cathéter 12 vers sa deuxième extrémité 122.

Selon une cinquième étape E5, de l'air est injecté par la deuxième source d'injection d'air. L'air traverse le deuxième tuyau d'arrivée d'air 17 et le troisième cathéter 13, qui est situé autour d'au moins une partie du deuxième cathéter 12. L'air injecté détache les microbulles 30 formées à la deuxième extrémité 122 du deuxième cathéter 12, les distribuant ainsi aux cellules à transfecter.

Une séquence de déplacement et de distribution de microbulles 30 au moyen d'un dispositif de sonoporation tel que décrit dans les paragraphes précédents est illustrée en figure 5b.

Selon une première étape E11, le support 70 est déplacé par l'unité de déplacement 71, 72 de sorte à placer le centre géométrique d'un logement 61 i de la plaque 60, la plaque 60 comportant n logements 61, entre le transducteur ultrasonore 50 et le dispositif 10 de distribution de microbulles 30 sur l'axe Z vertical.

Selon une deuxième étape E12, des ondes ultrasonores sont émises par le transducteur ultrasonore 50 de sorte à stimuler le centre du logement 61 i, pendant qu'une première proportion des microbulles 30 est distribuée dans le logement 61 par le dispositif 10 de distribution de microbulles 30. L'émission des ondes ultrasonores et la distribution des microbulles 30 est stoppée après une certaine durée.

En variante, la distribution des microbulles 30 peut être réalisée non pas simultanément mais indépendamment de l'émission des ondes ultrasonores, ou avec un décalage temporel par rapport à celle-ci.

Ces étapes E11 et E12 peuvent être répétées p fois pour ce logement 61 i, selon une séquence de déplacement et de distribution de microbulles 30 souhaitée : le support 70 est déplacé de sorte à placer une autre partie du logement 61 i (par exemple une extrémité haute, basse, droite ou gauche du logement 61) entre le transducteur ultrasonore 50 et le dispositif 10 de distribution de microbulles 30 sur l'axe Z vertical, puis des ondes ultrasonores sont émises pendant qu'une deuxième proportion des microbulles 30 est distribuée.

La proportion de microbulles 30 distribuée à l'étape E12 peut être inversement proportionnelle au nombre p de fois où les étapes E11 et E12 sont répétées. Par exemple, dans le cas où les étapes E11 et E12 sont répétées cinq fois selon une séquence de déplacement consistant à placer d'abord le centre, puis l'extrémité haute, puis l'extrémité droite, puis l'extrémité basse, et enfin l'extrémité gauche du logement 61 i entre le transducteur ultrasonore 50 et le dispositif 10 de distribution de microbulles 30 sur l'axe Z vertical, la proportion de microbulles 30 distribuée à chaque étape E12 peut correspondre à un cinquième de la quantité totale de microbulles 30 à distribuer dans le logement 61 i. Ainsi, à l'issue de cette séquence, l'ensemble de la quantité de microbulles 30 à distribuer dans le logement 61 i sera distribuée.

D'autres séquences de déplacement du logement 61 i sont bien entendu envisageables. Par exemple, le support 70 peut dans l'étape E11 être déplacé de sorte à placer non pas le centre mais une extrémité du logement 61 entre le transducteur ultrasonore 50 et le dispositif 10 de distribution de microbulles 30. La séquence de déplacement du logement 61 peut s'effectuer en spirale, ou suivant tout autre motif souhaité. Cette séquence peut être répétée plusieurs fois pour chaque logement 61. Le nombre p de déplacements du logement 61 peut également varier. Par exemple, dans le cas où la molécule active est un acide nucléique et où la quantité d'acide nucléique à distribuer dans le logement 61 i est de 1 gramme, celui-ci peut être distribué en répétant trois fois les étapes E11 et E12, et en distribuant à chacune des trois étapes E12 0,33 g d'acide nucléique.

En variante, l'intégralité des microbulles 30 peut être distribuée en une seule fois dans le logement 61, le support 70 déplaçant le logement 61 i de manière à positionner son centre entre le transducteur ultrasonore 40 et le dispositif 10 de distribution de microbulles 30 sur l'axe Z vertical, et l'intégralité des microbulles 30 étant distribuée pendant l'étape E12.

La séquence de déplacement et de distribution des microbulles 30 du logement 61 i est terminée lorsque les p répétitions des étapes E11 et E12 sont effectuées. Le support 70 est déplacé de sorte à positionner un logement 61 i + 1 de la plaque 60 entre le transducteur ultrasonore 40 et le dispositif 10 de distribution de microbulles 30 sur l'axe Z vertical. Une nouvelle séquence de déplacement et de distribution des microbulles 30 est alors effectuée. Cette nouvelle séquence de déplacement et de distribution des microbulles 30 peut consister en un nombre q de répétitions des étapes E11 et E12, q pouvant être égal ou différent de p. Ainsi, chaque logement 61 bénéficie d'une séquence spécifique de déplacement et de distribution des microbulles 30.

Cette séquence peut être répétée pour chacun des n logements 61 de la plaque 60, le support 70 étant déplacé de manière à placer successivement chaque logement 61 de la plaque 60 entre le dispositif 10 de distribution de microbulles 30 et le transducteur ultrasonore 50. Ainsi, l'opération de sonoporation peut être réalisée sur chacun des n logements 61 contenant les cellules à transfecter. En variante, cette séquence peut n'être effectué que pour certains des n logements 61 de la plaque 60.

Le procédé de sonoporation peut être exécuté par un produit programme d'ordinateur exécuté par un processeur. Un tel logiciel peut communiquer avec l'unité de traitement afin de contrôler de manière automatique les caractéristiques de la distribution des microbulles 30 (volume distribué, position et instant de distribution, temps entre chaque microbulle 30 distribuée, nombre de distributions, etc.), les paramètres des ondes ultrasonores (fréquence, amplitude, puissance, durée de transmission, etc.), le déplacement du support 70 (directions et vitesse de déplacement, position), les séquences de déplacement et de distribution des microbulles 30, l'ouverture et la fermeture du capot 83, etc. Ainsi, l'opération de sonoporation peut être réalisée de manière automatique, reproductible, avec des paramètres spécifiques à chaque logement 61 de la plaque 60. Le rendement d'une telle sonoporation est par conséquent amélioré par rapport aux rendements actuels.

La séquence de déplacement et de distribution des microbulles 30 peut être prédéterminée avant l'opération de sonoporation, auquel cas l'utilisateur peut la modifier au cours de l'opération via l'interface de l'unité de traitement. En variante, la séquence de déplacement et de distribution des microbulles 30 peut être choisie au cours de l'opération par l'utilisateur.

## Revendications

1. Dispositif (10) de distribution de microbulles (30) pour une sonoporation cellulaire, **caractérisé en ce que** le dispositif (10) comprend :
- un tube (14) symétrique autour d'un axe (Z) vertical, ledit tube (14) étant configuré pour recevoir un flacon (20) contenant une solution (26) de microbulles (30),
- au moins deux cathéters (11, 12) disposés à l'intérieur du tube (14), lesdits cathéters (11, 12) étant au moins en partie orientés de façon sensiblement parallèle à l'axe (Z) vertical, chacun des cathéters (11, 12) présentant en une première extrémité (111, 121) un orifice adapté pour déboucher dans le flacon (20) lorsque celui-ci est reçu par le tube (14), le premier cathéter (11) étant adapté pour être raccordé à une première source d'injection d'air, le deuxième cathéter (12) présentant une deuxième extrémité (122),
dans lequel, lorsque le premier cathéter (11) est raccordé à la première source d'injection d'air, de l'air peut être injecté dans le flacon (20) via l'orifice du premier cathéter (11), l'air injecté générant une surpression dans le flacon (20), de sorte que des microbulles (30) contenues dans le flacon (20) entrent dans le deuxième cathéter (12) via l'orifice de sa première extrémité (121), puis se déplacent à l'intérieur du deuxième cathéter (12) vers sa deuxième extrémité (122).

2. Dispositif (10) de distribution de microbulles (30) selon la revendication 1, comprenant en outre un troisième cathéter (13) au moins en partie situé autour d'au moins une partie du deuxième cathéter (12), le troisième cathéter (13) étant adapté pour être raccordé à une deuxième source d'injection d'air.

3. Dispositif de sonoporation cellulaire comprenant un dispositif (10) de distribution de microbulles (30) selon l'une des revendications 1 ou 2.

4. Dispositif de sonoporation selon la revendication 3, comprenant en outre une première source d'injection d'air raccordée au premier cathéter (11), et éventuellement une deuxième source d'injection d'air raccordée au troisième cathéter (13), lorsque le dispositif (10) de distribution de microbulles (30) comprend un troisième cathéter (13).

5. Dispositif de sonoporation cellulaire selon l'une des revendications 3 ou 4, comprenant en outre :
- une cuve (40) destinée à être remplie au moins partiellement d'un liquide,
- un transducteur ultrasonore (50) disposé à l'intérieur de la cuve (40),
- une plaque (60) comprenant au moins un logement (61) destiné à recevoir des cellules à transfecter,
- un support (70) mobile adapté pour recevoir la plaque (60),
- une unité de déplacement (71, 72) du support (70) mobile, configurée pour déplacer le support (70).

6. Dispositif de sonoporation selon la revendication 5, dans lequel l'unité de déplacement (71, 72) comprend des rails (71) s'étendant dans au moins deux dimensions définissant un plan perpendiculaire à l'axe (Z) vertical, dit plan horizontal, la troisième dimension étant celle de l'axe (Z) vertical, le support (70) étant destiné à être déplacé le long desdits rails (71).

7. Dispositif de sonoporation selon l'une des revendications 5 ou 6, dans lequel l'unité de déplacement (71, 72) comprend en outre un moteur adapté pour déplacer le support (70) dans au moins deux dimensions définissant un plan perpendiculaire à l'axe (Z) vertical, dit plan horizontal, la troisième dimension étant celle de l'axe (Z) vertical.

8. Dispositif de sonoporation selon l'une des revendications 5 à 7, dans lequel le dispositif (10) de distribution de microbulles (30) est positionné en face du transducteur ultrasonore (50) selon l'axe (Z) vertical.

9. Dispositif de sonoporation selon l'une des revendications 5 à 8, comprenant en outre un boîtier (80) comprenant une première partie (81) configurée pour loger les éventuelles sources d'injection d'air ainsi qu'au moins une partie de l'unité de déplacement (71, 72), le boîtier (80) comprenant en outre une deuxième partie (82) configurée pour loger le dispositif (10) de distribution de microbulles (30), la cuve (40), le transducteur ultrasonore (50), la plaque (60) et le support (70) mobile, la deuxième partie (82) comprenant en outre un capot (83) adapté pour être ouvert ou fermé, ledit capot (83) permettant en position ouverte à un utilisateur d'accéder au moins à la plaque (60).

10. Dispositif de sonoporation selon l'une des revendications 3 à 9, comprenant en outre une unité de traitement configurée pour contrôler au moins l'un des éléments suivants : dispositif (10) de distribution de microbulles (30), transducteur ultrasonore (50), unité de déplacement (71, 72), éventuelles première source d'injection d'air et deuxième source d'injection d'air, capot (83).

11. Procédé de sonoporation cellulaire au moyen d'un dispositif (10) de distribution de microbulles (30) selon l'une des revendications 1 ou 2, un flacon (20) contenant une solution (26) de microbulles (30) étant reçu dans le dispositif (10) de distribution de microbulles (30),
le procédé de sonoporation comprenant une étape d'injection d'air dans le flacon (20) via l'orifice de la première extrémité (111) du premier cathéter (11), l'air injecté générant une surpression dans le flacon (20), de sorte que des microbulles (30) contenues dans le flacon (20) entrent dans le deuxième cathéter (12) via l'orifice de sa première extrémité (121) puis se déplacent à l'intérieur du deuxième cathéter (12) vers sa deuxième extrémité (122).

12. Procédé de sonoporation selon la revendication 11, comprenant en outre une étape d'injection d'air dans un troisième cathéter (13) au moins en partie situé autour d'au moins une partie du deuxième cathéter (12).

13. Procédé de sonoporation selon l'une des revendications 11 et 12, comprenant en outre une étape de déplacement du support (70) dans laquelle, le dispositif (10) de distribution de microbulles (30) étant positionné en face du transducteur ultrasonore (50) selon l'axe (Z) vertical, le support (70) est déplacé de manière à placer successivement chaque logement (61) de la plaque (60) entre le dispositif (10) de distribution de microbulles (30) et le transducteur ultrasonore (50) selon l'axe (Z) vertical.

14. Produit programme d'ordinateur comprenant des instructions de code pour l'exécution d'un procédé de sonoporation selon l'une des revendications 11 à 13 lorsque ce programme est exécuté par un processeur.

## Patentansprüche

1. Vorrichtung (10) zur Verteilung von Mikrobläschen (30) für eine Zellsonoporation, **dadurch gekennzeichnet, dass** die Vorrichtung (10) umfasst:
- ein Rohr (14), das um eine vertikale Achse (Z) symmetrisch ist, wobei das Rohr (14) dazu ausgestaltet ist, ein Fläschchen (20) aufzunehmen, das eine Lösung (26) von Mikrobläschen (30) enthält,
- mindestens zwei Katheter (11, 12), die im Inneren des Rohrs (14) angeordnet sind, wobei die Katheter (11, 12) zumindest teilweise auf zur vertikalen Achse (Z) im Wesentlichen parallele Weise ausgerichtet sind, wobei jeder der Katheter (11, 12) an einem ersten Ende (111, 121) eine Öffnung aufweist, die dazu geeignet ist, in das Fläschchen (20) zu münden, wenn dieses von dem Rohr (14) aufgenommen wird, wobei der erste Katheter (11) dazu geeignet ist, an eine erste Lufteinpressquelle angeschlossen zu werden, wobei der zweite Katheter (12) ein zweites Ende (122) aufweist,
wobei, wenn der erste Katheter (11) an die erste Lufteinpressquelle angeschlossen ist, Luft über die Öffnung des ersten Katheters (11) in das Fläschchen (20) eingepresst werden kann, wobei die eingepresste Luft einen Überdruck in dem Fläschchen (20) erzeugt, derart dass Mikrobläschen (30), die in dem Fläschchen (20) enthalten sind, über die Öffnung seines ersten Endes (121) in den zweiten Katheter (12) eintreten und sich dann im Inneren des zweiten Katheters (12) hin zu seinem zweiten Ende (122) verlagern.

2. Vorrichtung (10) zur Verteilung von Mikrobläschen (30) nach Anspruch 1, die ferner einen dritten Katheter (13) umfasst, der sich zumindest teilweise um mindestens einen Teil des zweiten Katheters (12) befindet, wobei der dritte Katheter (13) dazu geeignet ist, an eine zweite Lufteinpressquelle angeschlossen zu werden.

3. Vorrichtung zur Zellsonoporation, die eine Vorrichtung (10) zur Verteilung von Mikrobläschen (30) nach einem der Ansprüche 1 oder 2 umfasst.

4. Sonoporationsvorrichtung nach Anspruch 3, die ferner eine erste Lufteinpressquelle, die an den ersten Katheter (11) angeschlossen ist, und gegebenenfalls eine zweite Lufteinpressquelle umfasst, die an den dritten Katheter (13) angeschlossen ist, wenn die Vorrichtung (10) zur Verteilung von Mikrobläschen (30) einen dritten Katheter (13) umfasst.

5. Vorrichtung zur Zellsonoporation nach einem der Ansprüche 3 oder 4, die ferner umfasst:
- einen Behälter (40), der dazu bestimmt ist, zumindest teilweise mit einer Flüssigkeit gefüllt zu werden,
- einen Ultraschallwandler (50), der im Inneren des Behälters (40) angeordnet ist,
- eine Platte (60), die mindestens eine Aufnahme (61) umfasst, die dazu bestimmt ist, zu transfizierende Zellen aufzunehmen,
- einen beweglichen Träger (70), der dazu geeignet ist, die Platte (60) aufzunehmen,
- eine Verlagerungseinheit (71, 72) des beweglichen Trägers (70), die dazu ausgestaltet ist, den Träger (70) zu verlagern.

6. Sonoporationsvorrichtung nach Anspruch 5, wobei die Verlagerungseinheit (71, 72) Schienen (71) umfasst, die sich in mindestens zwei Dimensionen erstrecken, die eine Ebene senkrecht zur vertikalen Achse (Z) definieren, die horizontale Ebene genannt wird, wobei die dritte Dimension diejenige der vertikalen Achse (Z) ist, wobei der Träger (70) dazu bestimmt ist, entlang der Schienen (71) verlagert zu werden.

7. Sonoporationsvorrichtung nach einem der Ansprüche 5 oder 6, wobei die Verlagerungseinheit (71, 72) ferner einen Motor umfasst, der dazu geeignet ist, den Träger (70) in mindestens zwei Dimensionen zu verlagern, die eine Ebene senkrecht zur vertikalen Achse (Z) definieren, die horizontale Ebene genannt wird, wobei die dritte Dimension diejenige der vertikalen Achse (Z) ist.

8. Sonoporationsvorrichtung nach einem der Ansprüche 5 bis 7, wobei die Vorrichtung (10) zur Verteilung von Mikrobläschen (30) dem Ultraschallwandler (50) entlang der vertikalen Achse (Z) gegenüberstehend positioniert ist.

9. Sonoporationsvorrichtung nach einem der Ansprüche 5 bis 8, die ferner ein Gehäuse (80) umfasst, das einen ersten Teil (81) umfasst, der dazu ausgestaltet ist, etwaige Lufteinpressquellen sowie mindestens einen Teil der Verlagerungseinheit (71, 72) unterzubringen, wobei das Gehäuse (80) ferner einen zweiten Teil (82) umfasst, der dazu ausgestaltet ist, die Vorrichtung (10) zur Verteilung von Mikrobläschen (30), den Behälter (40), den Ultraschallwandler (50), die Platte (60) und den beweglichen Träger (70) unterzubringen, wobei der zweite Teil (82) ferner eine Haube (83) umfasst, die dazu geeignet ist, geöffnet oder geschlossen zu sein, wobei die Haube (83) in der geöffneten Stellung einem Benutzer den Zugang zu mindestens der Platte (60) ermöglicht.

10. Sonoporationsvorrichtung nach einem der Ansprüche 3 bis 9, die ferner eine Verarbeitungseinheit umfasst, die dazu ausgestaltet ist, mindestens eines der folgenden Elemente zu steuern: Vorrichtung (10) zur Verteilung von Mikrobläschen (30), Ultraschallwandler (50), Verlagerungseinheit (71, 72), etwaige erste Lufteinpressquelle und zweite Lufteinpressquelle, Haube (83) .

11. Verfahren zur Zellsonoporation mittels einer Vorrichtung (10) zur Verteilung von Mikrobläschen (30) nach einem der Ansprüche 1 oder 2, wobei ein Fläschchen (20), das eine Lösung (26) von Mikrobläschen (30) enthält, in der Vorrichtung (10) zur Verteilung von Mikrobläschen (30) aufgenommen wird, wobei das Sonoporationsverfahren einen Schritt des Einpressens von Luft in das Fläschchen (20) über die Öffnung des ersten Endes (111) des ersten Katheters (11) umfasst, wobei die eingepresste Luft einen Überdruck in dem Fläschchen (20) erzeugt, derart dass Mikrobläschen (30), die in dem Fläschchen (20) enthalten sind, in den zweiten Katheter (12) über die Öffnung seines ersten Endes (121) eintreten und sich dann im Inneren des zweiten Katheters (12) hin zu seinem zweiten Ende (122) verlagern.

12. Sonoporationsverfahren nach Anspruch 11, das ferner einen Schritt des Einpressens von Luft in einen dritten Katheter (13) mittels eines Teils umfasst, der sich um mindestens einen Teil des zweiten Katheters (12) befindet.

13. Sonoporationsverfahren nach einem der Ansprüche 11 und 12, das ferner einen Schritt des Verlagerns des Trägers (70) umfasst, in dem die Vorrichtung (10) zur Verteilung von Mikrobläschen (30) dem Ultraschallwandler (50) entlang der vertikalen Achse (Z) gegenüberstehend positioniert ist, der Träger (70) derart verlagert wird, dass jede Aufnahme (61) der Platte (60) aufeinanderfolgend entlang der vertikalen Achse (Z) zwischen der Vorrichtung (10) zur Verteilung von Mikrobläschen (30) und dem Ultraschallwandler (50) platziert wird.

14. Computerprogrammprodukt, das Codeanweisungen für die Ausführung eines Sonoporationsverfahrens nach einem der Ansprüche 11 bis 13 umfasst, wenn dieses Programm durch einen Prozessor ausgeführt wird.

## Claims

1. A device (10) for dispensing microbubbles (30) for cell sonoporation, **characterized in that** the device (10) comprises:
- a tube (14) symmetrical about a vertical axis (Z), said tube (14) being configured to receive a vial (20) containing a solution (26) of microbubbles (30),
- at least two catheters (11, 12) arranged inside the tube (14), said catheters (11, 12) being at least partially oriented substantially parallel to the vertical axis (Z), each of the catheters (11, 12) having at a first end (111, 121) an orifice adapted to open into the vial (20) when the latter is received by the tube (14), the first catheter (11) being adapted to be connected to a first air injection source, the second catheter (12) having a second end (122),
wherein, when the first catheter (11) is connected to the first air injection source, air can be injected into the vial (20) via the orifice of the first catheter (11), the injected air generating an overpressure in the vial (20), so that microbubbles (30) contained in the vial (20) enter the second catheter (12) via the orifice of its first end (121), and then move inside the second catheter (12) toward its second end (122).

2. The device (10) for dispensing microbubbles (30) as claimed in claim 1, further comprising a third catheter (13) at least partially located around at least part of the second catheter (12), the third catheter (13) being adapted to be connected to a second air injection source.

3. A cell sonoporation device comprising a device (10) for dispensing microbubbles (30) as claimed in one of claims 1 or 2.

4. The sonoporation device as claimed in claim 3, further comprising a first air injection source connected to the first catheter (11), and optionally a second air injection source connected to the third catheter (13), when the device (10) for dispensing microbubbles (30) comprises a third catheter (13).

5. The cell sonoporation device as claimed in one of claims 3 or 4, further comprising:
- a tank (40) intended to be at least partially filled with a liquid,
- an ultrasonic transducer (50) arranged inside the tank (40),
- a plate (60) comprising at least one receptacle (61) intended to receive cells to be transfected,
- a mobile support (70) adapted to receive the plate (60),
- a mobile support (70) moving unit (71, 72), configured to move the support (70).

6. The cell sonoporation device as claimed in claim 5, wherein the moving unit (71, 72) comprises rails (71) extending in at least two dimensions defining a plane perpendicular to the vertical axis (Z), called horizontal plane, the third dimension being that of the vertical axis (Z), the support (70) being intended to be moved along said rails (71).

7. The sonoporation device as claimed in one of claims 5 or 6, wherein the moving unit (71, 72) further comprises a motor adapted to move the support (70) in at least two dimensions defining a plane perpendicular to the vertical axis (Z), called horizontal plane, the third dimension being that of the vertical axis (Z).

8. The sonoporation device as claimed in one of claims 5 to 7, wherein the device (10) for dispensing microbubbles (30) is positioned facing the ultrasonic transducer (50) along the vertical axis (Z).

9. The sonoporation device as claimed in one of claims 5 to 8, further comprising a housing (80) comprising a first part (81) configured to house the possible air injection sources as well as at least part of the moving unit (71, 72), the housing (80) further comprising a second part (82) configured to house the device (10) for dispensing microbubbles (30), the tank (40), the ultrasonic transducer (50), the plate (60) and the mobile support (70), the second part (82) further comprising a cover (83) adapted to be opened or closed, said cover (83) in the open position allowing a user to access at least the plate (60).

10. The sonoporation device as claimed in one of claims 3 to 9, further comprising a processing unit configured to control at least one of the following elements: device (10) for dispensing microbubbles (30), ultrasonic transducer (50), moving unit (71, 72), possible first air injection source and second air injection source, cover (83).

11. **A** process for cell sonoporation by means of a device (10) for dispensing microbubbles (30) as claimed in one of claims 1 or 2, a vial (20) containing a solution (26) of microbubbles (30) being received in the device (10) for dispensing microbubbles (30),
the sonoporation process comprising a step of injecting air into the vial (20) via the orifice of the first end (111) of the first catheter (11), the injected air generating an overpressure in the vial (20), so that microbubbles (30) contained in the vial (20) enter the second catheter (12) via the orifice of its first end (121) and then move inside the second catheter (12) toward its second end (122).

12. The sonoporation process as claimed in claim 11, further comprising a step of injecting air into a third catheter (13) at least partially located around at least part of the second catheter (12).

13. The sonoporation process as claimed in one of claims 11 and 12, further comprising a step of moving the support (70) wherein, the device (10) for dispensing microbubbles (30) being positioned facing the ultrasonic transducer (50) along the vertical axis (Z), the support (70) is moved so as to place each receptacle (61) of the plate (60) successively between the device (10) for dispensing microbubbles (30) and the ultrasonic transducer (50) along the vertical axis (Z).

14. A computer program product comprising code instructions for the execution of a sonoporation process as claimed in one of claims 11 to 13 when this program is executed by a processor.
